# EUROPEAN PATENT APPLICATION

(11) **EP 4 834 899 A1**
(43) Date of publication of application: **04.11.2026**
(21) Application number: 24910603.0
(22) Date of filing: 04.12.2024
(51) Int. Cl.: C07D 413/10, A01N 43/80, A01P 13/00

(54) **NEW CRYSTAL FORM OF ISOXAZOLINE URACIL COMPOUND, PREPARATION METHOD, AND HERBICIDE AND USE**

(30) Priority: 28.12.2023 CN 202311851631
(71) Applicant: Nantong Jiangshan Agrochemical & Chemicals Co., Ltd., Nantongshi, Jiangsu 226000 (CN); Jiangshan (Yichang) Crop Technology Co., Ltd., Zhijiang City Yichang, Hubei 443200 (CN)
(72) Inventor: WANG, Li, Nantong, Jiangsu 226000 (CN); DONG, Lei, Nantong, Jiangsu 226000 (CN); DU, Hui, Nantong, Jiangsu 226000 (CN); XU, Wei, Nantong, Jiangsu 226000 (CN)
(74) Representative: Cabinet Chaillot
(86) International application number: PCT/CN2024/136893
(87) International publication number: WO 2025/139671

(57) **Abstract**

A new crystal form of an isoxazoline uracil compound represented by formula (1), a preparation method, and an herbicide containing the new crystal form of an isoxazoline uracil compound and the use thereof. An X-ray powder diffraction pattern of the new crystal form has characteristic peaks at diffraction angles 2θ of 6.3 ± 0.2, 7.2 ± 0.2, 11.2 ± 0.2, 14.3 ± 0.2, 17.3 ± 0.2, 19.6 ± 0.2, 20.7 ± 0.2, 24.3 ± 0.2, 25.2 ± 0.2, and 26.1 ± 0.2. The new crystal form solves the problems of pipeline blockage during production, difficulty in acquiring materials, inaccurate metering, and difficulty in storage and sub-packaging caused by oily isoxazoline uracil compounds in the prior art. In formula (1), R1 is fluorine, R2 is chlorine, R3 and R4 are hydrogen, R5 is CO₂C₂H₅, and R6 is methyl.

## Description

### FIELD OF TECHNOLOGY

The present invention relates to the technical field of C07D413/10, and more specifically, the present invention relates to a novel crystal form of an isoxazoline uracil compound, a preparation method therefor, a herbicide, and use thereof.

### BACKGROUND

Isoxazoline uracil compounds can wipe off weeds of soybeans, cotton, vegetables, maize, peanuts, and the like in the process of growth. Currently, the prepared isoxazoline uracil compounds are all oily matters; for example, a Chinese patent CN201811146442 discloses that compound II is reacted with methyl chloride to obtain an oily isoxazoline-containing uracil compound; moreover, the last step of obtaining the product is completed in a way of distilling off solvent; such a way has a certain difficulty in expansion, in particular to scale-up production. Particularly, the product is an oily matter and the solvent is hard to be thoroughly dried by distillation; moreover, the oily matter cannot be dried; hence, the content of the product can be not up to the requirements. In addition, the oily matter may be coagulated at low temperature in production due to viscosity, resulting in a series of problems such as pipe blockage of production equipment, difficulties in discharging, difficulties in metering, difficulties in taking materials, difficulties in subpackaging, difficulties in transportation, and difficulties in dissolving.

### SUMMARY

Directed to some problems existing in the prior art, a first aspect of the present invention is to provide a novel crystal form of an isoxazoline uracil compound; its powder X-ray diffraction pattern has characteristic peaks at a diffraction angle 2θ of 6.3±0.2, 7.2±0.2, 11.2±0.2, 14.3±0.2, 17.3±0.2, 19.6±0.2, 20.7±0.2, 24.3±0.2, 25.2±0.2, and 26.1±0.2,
where, the isoxazoline uracil compound is shown in the following formula (1), 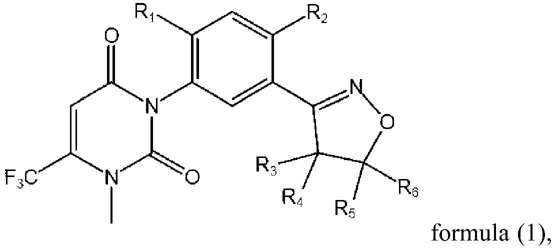 R₁ is fluorine, R₂ is chlorine, R₃ and R₄ are hydrogen, R₅ is CO₂C₂H₅, and R₆ is methyl.

In an embodiment, the powder X-ray diffraction pattern thereof further has characteristic peaks at a diffraction angle 2θ of 27.7±0.2, 29.3±0.2, 30.3±0.2, 31.8±0.2, 33.2±0.2.

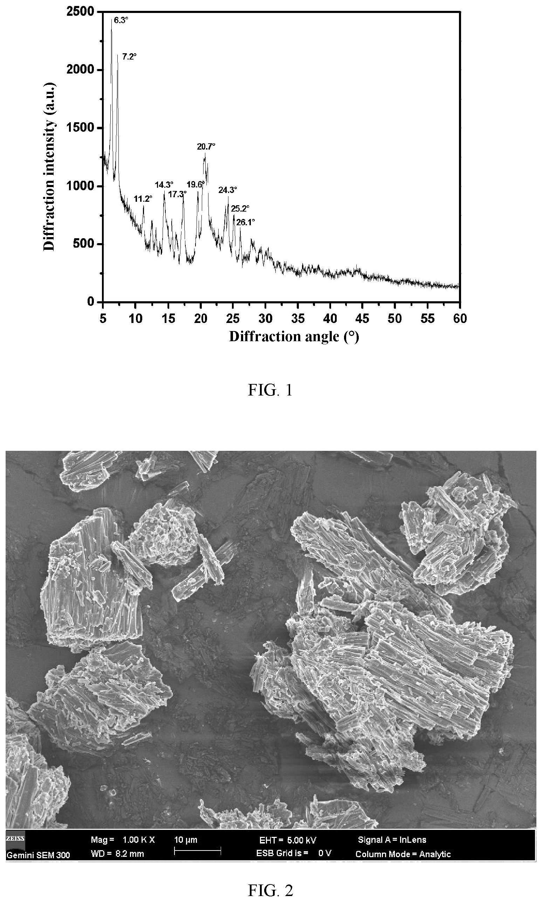

In an embodiment, the isoxazoline uracil compound has characteristic diffraction peaks in the X-ray diffraction (XRD) spectrum as shown in FIG. 1.

Isoxazoline uracil compounds are oily sticky matters due to specific properties in the prior art. However, in product production, oily sticky materials may cause low-temperature coagulation of materials, pipe blockage of production equipment, and difficulties in discharging materials. Meanwhile, when the oily sticky material is used as a herbicide, there possibly exist difficulties in taking materials, difficulties in metering accurately, and difficulties in feeding. Moreover, the solvent is hard to be dried by distillation and cannot be dried, and thus, product content can be not up to the requirements. Moreover, the oily sticky material is free of a fixed form during transportation and storage and thus, is hard to be subpackaged. In use procedure, the oily sticky material is sticky when it is dissolved into a solvent and thus, prone to be attached onto an agitator blade, resulting in a slow dissolution velocity. A novel crystal form is unexpectedly found by the applicant in experiments. The novel compound has characteristic peaks at a diffraction angle 2θ of 6.3±0.2, 7.2±0.2, 11.2±0.2, 14.3±0.2, 17.3±0.2, 19.6±0.2, 20.7±0.2, 24.3±0.2, 25.2±0.2, and 26.1±0.2; the crystal form of the isoxazoline uracil compound makes it being in a powdery solid state, thus solving a series of problems of the oily matter caused by the crystal form in the prior art.

In an embodiment, as shown in FIG. 2, the novel crystal form is a bunchy crystal or block crystal aggregated by rodlike crystals.

Preferably, the rodlike crystal has a length of 100 nm-10 mm.

It is unexpectedly found by the applicant that when the crystal form of the isoxazoline uracil compound is a bunchy crystal or block crystal aggregated by rodlike crystals, and particularly, the rodlike crystal has a thickness of 100 nm-10 mm; at this time, the isoxazoline uracil compound is not only kept in a form of powdery solid. Meanwhile, in the process of producing formulations, the powdery solid is more readily dispersed and dissolved. The applicant believes that the possible reason is as follows: rodlike crystals between the bunchy crystal or block crystal are arranged orderly; under the action of an organic or inorganic solvent, liquidity is promoted; meanwhile, a solvent is readily diffused between the rodlike crystals to enhance the dispersion between the rodlike crystals.

In an embodiment, the infrared spectrogram shows characteristic absorption peaks at wave numbers of 458±2cm⁻¹, 516±2cm⁻¹, 565±2cm⁻¹, 603±2cm⁻¹, 632±2cm⁻¹, 674±2cm⁻¹, 715±2cm⁻¹, 746±2cm⁻¹, 781±2cm⁻¹, 985±2cm⁻¹, 1066±2cm⁻¹, 1402±2cm⁻¹, 1629±2cm⁻¹, and 3239±2cm⁻¹.

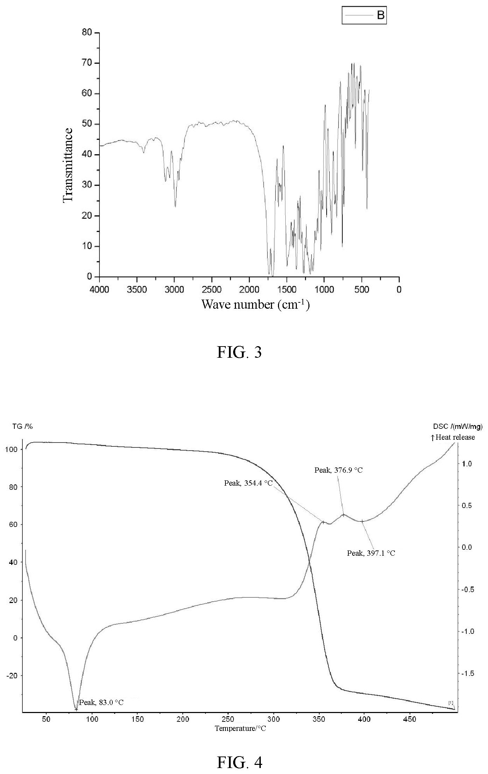

Preferably, the novel crystal form of the isoxazoline uracil compound has characteristic diffraction peaks in the XRD spectrum as shown in FIG. 3.

In an embodiment, an H NMR spectrum thereof has the following peaks: ¹H NMR (400 MHz, CDCl₃) δ 7.67 (d, *J* = 7.6 Hz, 1H), 7.35 (d, *J* = 9.0 Hz, 1H), 6.35 (s, 1H), 4.26 (q, *J* = 7.1 Hz, 2H), 4.00 (t, *J* = 16.8 Hz, 1H), 3.54 (s, 3H), 3.39 (dd, *J* = 17.4, 13.9 Hz, 1H), 1.70 (d, *J* = 1.4 Hz, 3H), 1.32 (t, *J* = 7.1 Hz, 3H).

In an embodiment, a C NMR spectrum thereof has the following peaks: ¹³C NMR (101 MHz, CDCl₃) δ 171.57 (s), 159.74 (s), 159.33 (s), 156.76 (s), 154.43 (s), 150.52 (s), 141.83 (q, *J* = 34.7 Hz), 134.74 (d, *J* = 9.8 Hz), 131.90 (d, *J* = 1.2 Hz), 125.87 (d, *J* = 4.0 Hz), 121.40 (d, *J* = 14.0 Hz), 119.35 (q, *J* = 276.7 Hz),119.30 (s), 119.07 (s), 102.98 (q, *J* = 5.5 Hz), 87.13 (d, *J* = 1.7 Hz), 62.10 (s), 46.53 (s), 32.69 (q, *J* = 3.6 Hz), 23.28 (s), 14.06 (s).

In an embodiment, the differential scanning calorimetry (DSC) spectrum thereof has an absorption peak at 360-390°C and an exothermic peak at 60-100°C.

Preferably, the novel crystal form has absorption and exothermic peaks in the DSC spectrum as shown in FIG. 4.

It is unexpectedly found by the applicant that as shown in the absorption and exothermic peaks of FIG. 4 of the present application, the novel crystal form of the isoxazoline uracil compound enables the isoxazoline uracil compound to have better storage stability. Compared with the oily isoxazoline uracil compounds in the prior art, the novel crystal form has significantly improved advantages. This is probably because the molecular arrangement of the isoxazoline uracil compound is relatively regular to improve heat resistance; moreover, molecules thereof can be further dispersed rapidly under the action of an organic solvent or water in certain content.

Test conditions for the XRD spectrum of the novel crystal form of the isoxazoline uracil compound in the present application are as follows: absorption coefficient = 0; activating absorber = 0; α1 = 1.5406; α2 = 1.54439; α mean value = 1.54184; α ratio = 0.5; analyzer = 0; anode = copper; anti-scattering slit = 6.9; beam optical mark = 189; β=1.39222; β relative intensity = 0; deactivating absorber = 0; detector slit = 12.21; divergence slit = 0.6; goniometer control = 9999; goniometer diameter = 500; goniometer type = 516; goniometer grade = 0; monochromator = 0; near sample slit = 0; major Soller slit = 2.5; sample converter = 0; second Soller slit = 2.5; synchronous shaft = 0; film adhesion = 0; wave unit = A; actually used λ = 1.5406; additional detector shield = 257; data record length = 4; delay time = 0; display plane No. = 0; estimated scan time = 0; extra parameter mask = 0; generator current = 40; generator voltage = 40; increment = 0.0194797; ascending 3 = 0; number of completed data = 2824; count data = 1; number of detectors = 0; number of drivers = 2; number of encoder driver = 0; number of measured data = 2824; number of variable parameters = 0; range start time = 1088421888; revolving speed = 0; scan mode = 1; scan type = locked coupling; simulate Meas condition = 0; slit switcher inch = 9999; Smooting width = 0; start = 5; step number = 2824; synchronous rotation = 0; and time = 19.2.

Test conditions for the DSC spectrum: instrument: NETZSCH STA 449F3; sample: 1; sample mass/mg: 8; material: null; reference mass/mg: 0; crucible type: DSC/TG pan Al₂O₃; sample crucible mass/mg: 0; sample crucible mass/mg: 0; code correction: 20; EXO: 1; range: 30/15.0 (K/min)/500; segment: S1/1.

Test conditions for scanning electron microscopy (SEM): Gemini SEM 300, Mag=1.00KX, WD=8.2mm, EHT=5.00kV, ESB Grid is=0V, Signal A=lnLens, Column Mode=Analytic.

In an embodiment, the preparation method of the novel crystal form includes: 3-(2-chloro-4-fluoro-5-(2,6-dioxo-4-trifluoromethyl-3,6-dihydropyrimidin-1(2H)-yl)phenyl)-5-methyl-4,5-dihydroisoxazole-5-eth yl carboxylate is reacted with methyl chloride in toluene; at the end of the reaction, the resulting product is washed with water to remove toluene, dropwise added with a solvent for dissolving, heated, cooled and recrystallized to obtain the final product.

According to the preparation method of the novel crystal form in the present application, those skilled in the art can make a conventional selection to obtain the novel crystal form in the present application.

A second aspect of the present invention is to provide a herbicide, including the novel crystal form of the isoxazoline uracil compound, and further including one or more pharmaceutically acceptable carriers and/or adjuvants.

A third aspect of the present invention is to provide use of the herbicide in controlling *Setaria viridis*, *Echinochloa crusgalli*, *Arthraxon hispidus*, *Cyperus difformis*, *Digitaria sanguinalis*, and *Cyperus serotinus.*

Compared with the prior art, the present invention has the following advantageous effects:

The present invention provides a novel crystal form of an isoxazoline uracil compound; the novel crystal form solves the problems of pipe blockage, difficulties in material taking, inaccurate measurement, difficulties in storage and subpackaging during production caused by oily isoxazoline uracil compounds in the prior art.

### BRIEF DESCRIPTION OF THE DRAWINGS

FIG. 1 is an XRD spectrum showing a novel crystal form of an isoxazoline uracil compound, i.e., Isoxafenacil according to the present invention;
FIG. 2 is an SEM spectrum showing the novel crystal form of the isoxazoline uracil compound, i.e., Isoxafenacil according to the present invention;
FIG. 3 is an infrared spectrogram showing the novel crystal form of the isoxazoline uracil compound, i.e., Isoxafenacil according to the present invention;
FIG. 4 is a DSC spectrum showing the novel crystal form of the isoxazoline uracil compound, i.e., Isoxafenacil according to the present invention;
FIG. 5 is an H nuclear magnetic resonance (H NMR) spectrum showing the novel crystal form of the isoxazoline uracil compound, i.e., Isoxafenacil according to the present invention; and
FIG. 6 is a C nuclear magnetic resonance (C NMR) spectrum showing the novel crystal form of the isoxazoline uracil compound, i.e., Isoxafenacil according to the present invention.

### DESCRIPTION OF THE EMBODIMENTS

The present invention will be specified by detailed embodiments below, but is not limited to the following detailed embodiments.

### Example 1

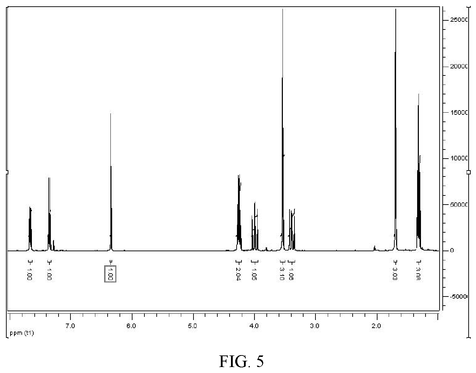

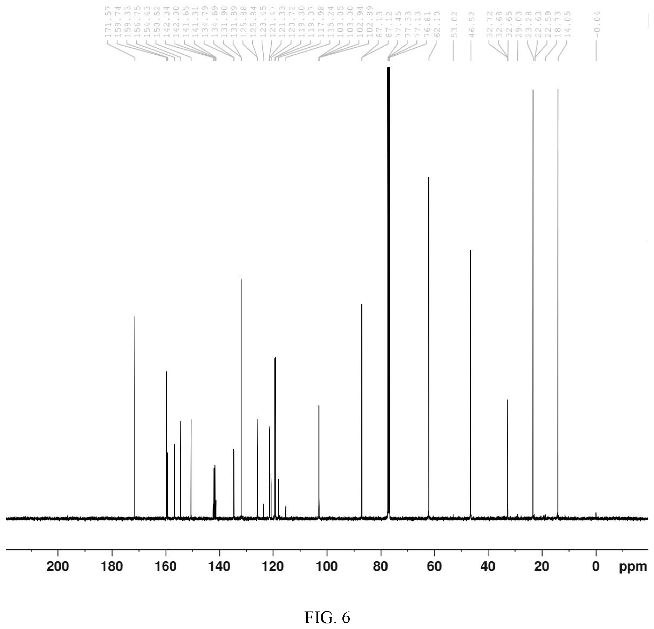

The novel crystal form of an isoxazoline uracil compound (Isoxafenacil, (3-(2-chloro-4-fluoro-5-(3-methyl-2,6-dioxo-4-trifluoromethyl-3,6-dihydropyrimidine-1(2H)-yl)phenyl)-5-methyl-4,5-dihydroisooxazole-5-ethyl carboxylate)) is shown in FIG. 1, and its powder X-ray diffraction pattern thereof has characteristic peaks at a diffraction angle 2θ of 6.3±0.2, 7.2±0.2, 11.2±0.2, 14.3±0.2, 17.3±0.2, 19.6±0.2, 20.7±0.2, 24.3±0.2, 25.2±0.2, and 26.1±0.2. As shown in FIG. 2, the novel crystal form is a bunchy crystal or block crystal aggregated by rodlike crystals, and the rodlike crystal has a length of 100 nm-10 mm. As shown in FIG. 3, the infrared spectrogram shows characteristic absorption peaks at wave numbers of 458±2cm⁻¹, 516±2cm⁻¹, 565±2cm⁻¹, 603±2cm⁻¹, 632±2cm⁻¹, 674±2cm⁻¹, 715±2cm⁻¹, 746±2cm⁻¹, 781±2cm⁻¹, 985±2cm⁻¹, 1066±2cm⁻¹, 1402±2cm⁻¹, 1629±2cm⁻¹, and 3239±2cm⁻¹. As shown in FIG. 4, the DSC spectrum shows absorption peaks at 360-390°C and exothermic peaks at 60-100°C. As shown in FIG. 5, the H NMR spectrum thereof has the following peaks: ¹H NMR (400 MHz, CDCl₃) δ 7.67 (d, *J* = 7.6 Hz, 1H), 7.35 (d, *J* = 9.0 Hz, 1H), 6.35 (s, 1H), 4.26 (q, *J* = 7.1 Hz, 2H), 4.00 (t, *J* = 16.8 Hz, 1H), 3.54 (s, 3H), 3.39 (dd, *J* = 17.4, 13.9 Hz, 1H), 1.70 (d, *J* = 1.4 Hz, 3H), 1.32 (t, *J* = 7.1 Hz, 3H). As shown in FIG. 6, the C NMR spectrum thereof has the following peaks: ¹³C NMR (101 MHz, CDCl₃) δ 171.57 (s), 159.74 (s), 159.33 (s), 156.76 (s), 154.43 (s), 150.52 (s), 141.83 (q, *J* = 34.7 Hz), 134.74 (d, *J* = 9.8 Hz), 131.90 (d, *J* = 1.2 Hz), 125.87 (d, *J* = 4.0 Hz), 121.40 (d, *J* = 14.0 Hz), 119.35 (q, *J* = 276.7 Hz),119.30 (s), 119.07 (s), 102.98 (q, *J* = 5.5 Hz), 87.13 (d, *J* = 1.7 Hz), 62.10 (s), 46.53 (s), 32.69 (q, *J* = 3.6 Hz), 23.28 (s), 14.06 (s).

The preparation method is as follows: 3-(2-chloro-4-fluoro-5-(2,6-dioxo-4-trifluoromethyl-3,6-dihydropyrimidin-1(2H)-yl)phenyl)-5-methyl-4,5-dihydroisoxazole-5-ethyl carboxylate was completely reacted with methyl chloride in toluene; the resulting product was washed with water to remove toluene, dropwise added with a solvent for dissolving, heated, then cooled and recrystallized to obtain the final product.

## Claims

1. A novel crystal form of an isoxazoline uracil compound, wherein a powder X-ray diffraction pattern thereof has characteristic peaks at a diffraction angle 2θ of 6.3±0.2, 7.2±0.2, 11.2±0.2, 14.3±0.2, 17.3±0.2, 19.6±0.2, 20.7±0.2, 24.3±0.2, 25.2±0.2, and 26.1±0.2,
wherein the isoxazoline uracil compound is shown in the following formula (1), 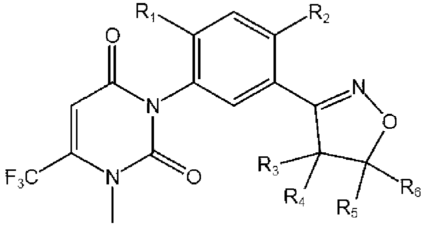
formula (1), wherein R₁ is fluorine, R₂ is chlorine, R₃ and R₄ are hydrogen, R₅ is CO₂C₂H₅, and R₆ is methyl.

2. The novel crystal form of an isoxazoline uracil compound according to claim 1, wherein the powder X-ray diffraction pattern thereof further has characteristic peaks at a diffraction angle 2θ of 27.7±0.2, 29.3±0.2, 30.3±0.2, 31.8±0.2, 33.2±0.2.

3. The novel crystal form of an isoxazoline uracil compound according to claim 1, wherein the novel crystal form is a bunchy crystal or block crystal aggregated by a rodlike crystal.

4. The novel crystal form of an isoxazoline uracil compound according to claim 3, wherein the rodlike crystal has a length of 100 nm-10 mm.

5. The novel crystal form of an isoxazoline uracil compound according to any one of claims 1-4, wherein a C nuclear magnetic resonance spectrum thereof has the following peaks: ¹³C NMR (101 MHz, CDCl₃) δ 171.57 (s), 159.74 (s), 159.33 (s), 156.76 (s), 154.43 (s), 150.52 (s), 141.83 (q, *J =* 34.7 Hz), 134.74 (d, *J =* 9.8 Hz), 131.90 (d, *J=* 1.2 Hz), 125.87 (d, *J* = 4.0 Hz), 121.40 (d, *J =* 14.0 Hz), 119.35 (q, *J =* 276.7 Hz),119.30 (s), 119.07 (s), 102.98 (q, *J=* 5.5 Hz), 87.13 (d, *J=* 1.7 Hz), 62.10 (s), 46.53 (s), 32.69 (q, *J=* 3.6 Hz), 23.28 (s), 14.06 (s).

6. The novel crystal form of an isoxazoline uracil compound according to claim 5, wherein an H nuclear magnetic resonance spectrum thereof has the following peaks: ¹H NMR (400 MHz, CDCl₃) δ 7.67 (d, *J* = 7.6 Hz, 1H), 7.35 (d, *J* = 9.0 Hz, 1H), 6.35 (s, 1H), 4.26 (q, *J* = 7.1 Hz, 2H), 4.00 (t, *J* = 16.8 Hz, 1H), 3.54 (s, 3H), 3.39 (dd, *J* = 17.4, 13.9 Hz, 1H), 1.70 (d, *J* = 1.4 Hz, 3H), 1.32 (t, *J* = 7.1 Hz, 3H).

7. The novel crystal form of an isoxazoline uracil compound according to claim 1, wherein a differential scanning calorimetry (DSC) spectrum thereof has an absorption peak at 360-390°C and an exothermic peak at 60-100°C.

8. A method for preparing the novel crystal form of an isoxazoline uracil compound according to any one of claims 1-7, comprising: completely reacting 3-(2-chloro-5-(2,6-dioxo-4-trifluoromethyl-3,6-dihydropyrimidine-1(2H)-yl)-4-fluorophenyl) -5-methyl-4,5-dihydroisoxazole-5-ethyl carboxylate with methyl chloride in toluene, then washing with water to remove toluene, dropwise adding a solvent for dissolving, heating and cooling for recrystallization to obtain the novel crystal.

9. A herbicide, comprising the novel crystal form of an isoxazoline uracil compound according to any one of claims 1-7, further comprising one or more pharmaceutically acceptable carriers and/or adjuvants.

10. Use of the herbicide according to claim 9 in controlling *Setaria viridis*, *Echinochloa crusgalli*, *Arthraxon hispidus*, *Cyperus difformis*, *Digitaria sanguinalis*, and *Cyperus serotinus.*
